(19) Europäisches Patentamt
European Patent Office
Office européen des brevets

(11) Publication number: **0 537 575 A1**

(12) **EUROPEAN PATENT APPLICATION**

(21) Application number: **92116898.5**

(22) Date of filing: **02.10.92**

(51) Int. Cl.[5]: **C07D 487/04**, A61K 31/495, //(C07D487/04,209:00,209:00)

(30) Priority: **07.10.91 JP 259188/91**

(43) Date of publication of application: **21.04.93 Bulletin 93/16**

(84) Designated Contracting States: **DE FR GB IT**

(71) Applicant: **KYOWA HAKKO KOGYO CO., LTD.**
**6-1, Ohtemachi 1-chome**
**Chiyoda-ku Tokyo-to(JP)**

(72) Inventor: **Nagamura, Satoru**
**512-1, Minamiisshiki, Nagaizumi-cho**
**Sunto-gun, Shizuoka-ken(JP)**
Inventor: **Saito, Hiromitsu**
**5-15-23, Tokura**
**Mishima-shi, Shizuoka-ken(JP)**
Inventor: **Hayakawa, Eiji**
**495-15, Chabatake**
**Sosono-shi, Shizuoka-ken(JP)**
Inventor: **Kato, Yasuki**
**2-8-7, Senpukugaoka**
**Sosono-shi, Shizuoka-ken(JP)**
Inventor: **Naganuma, Hirotake**
**198-1, Kakita, Shimizu-cho**
**Sunto-gun, Shizuoka-ken(JP)**

(74) Representative: **Casalonga, Axel et al**
**BUREAU D.A. CASALONGA - JOSSE**
**Morassistrasse 8**
**W-8000 München 5 (DE)**

(54) **Hydrobromide of DC-89 derivative having antitumor activity.**

(57) A hydrobromide of DC-89 derivative represented by formula (I):

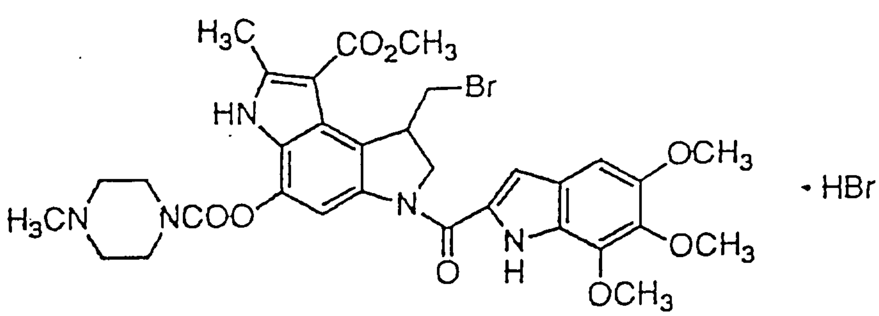

(I)

which has a strong anti-tumor activity and a high stability in the process for producing preparations and is useful as anti-tumor agents.

EP 0 537 575 A1

Background of the Invention

The present invention relates to a hydrobromide of DC-89 derivative. The compound has excellent anti-tumor activity and is expected to be useful as an anti-tumor agent.

There has been known a DC-89 derivative represented by formula (II):

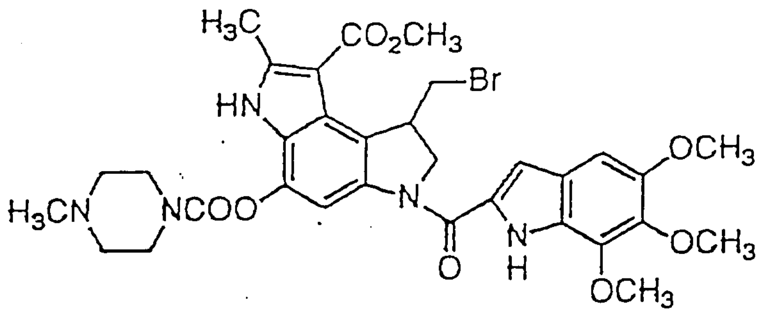

(II)

[hereinafter, referred to as Compound (II)] which shows an anti-tumor activity. Hydrochloride of Compound (II) [hereinafter, referred to as Compound (III)] is the only example of the salt of Compound (II) so far disclosed. (U.S. Patent No. 5,070,092).

Compound (III) is expected to be useful for injection. However, in view of its instability in a solution for producing a freezedried preparation for injection, there has been a constant demand for compounds having such a high stability in the process for producing preparations.

To obtain compounds which are superior to Compound (III) in stability, the salts of Compound (II) were synthesized using various inorganic acids or organic acids, etc., and the stability thereof has been examined. As a result, it has now been found that a hydrobromide of Compound (II) has a high stability and thus the present invention was completed.

Summary of the Invention

The present invention relates to a compound represented by formula (I) [hereinafter referred to as Compound (I)]:

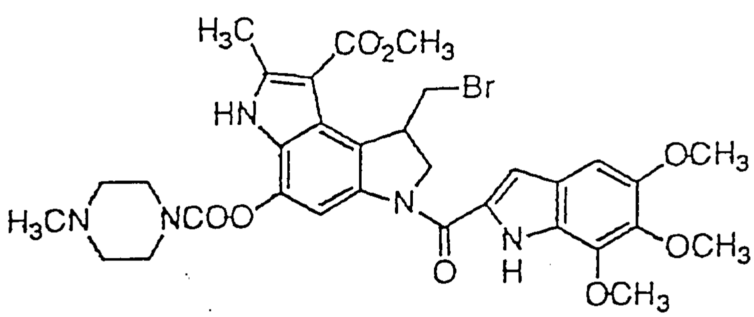

• HBr (I)

Detailed Description of the Invention

The process for preparing Compound (I) is described below.

Compound (I) can be obtained by allowing Compound (II) to react with 48% hydrobromic acid in an inert solvent.

As the inert solvent, methanol, ethanol, isopropanol, acetone, methylene chloride, chloroform, tetrahydrofuran, ethyl acetate, toluene, etc. may be used singly or in combination.

Hydrobromic acid is used in an amount of 1 to 3 equivalents based on Compound (II). The reaction is carried out at -30 ° C to 50 ° C and is completed in 30 minutes to 10 hours.

After completion of the reaction, the precipitate formed is separated by filtration, dried and then the desired compound is isolated and purified.

The pharmaceutical activities of Compound (I) are described below by referring to Experimental Example 1.

Experimental Example 1

The pharmaceutical activities of Compound (I) are shown below.

Growth Inhibition Test on $HeLaS_3$ Cells:

$HeLaS_3$ cells suspended in MEM medium containing 10% fetal calf serum and 2 mM glutamine (hereinafter referred to as medium A) to a concentration of $3 \times 10^4$ cells/ml were put into wells of a 96-well microtiter plate in an amount of 0.1 ml per well.

After culturing at 37°C overnight in a $CO_2$-incubator, 0.05 ml of a test sample appropriately diluted with medium A was added to each well.

The cells were further cultured at 37°C for 72 hours in the $CO_2$-incubator and the culture supernatant was removed. The residue was washed once with phosphate buffered physiological saline (PBS), and 0.1 ml of a medium containing medium A and 0.02% Neutral Red was added to each well, followed by culturing at 37°C for one hour in the $CO_2$-incubator to stain the cells. After removal of the culture supernatant, the cells were washed once with physiological saline, and the dye was extracted with 0.001N HCl/30% ethanol. Absorbance of the extract at 550 nm was measured with a microplate reader. By comparing the absorbance of the extract of intact cells with those of the cells treated with the test compound at known concentrations, the concentration of the test compound which inhibited growth of the cells by 50% ($IC_{50}$) was determined.

The results are shown in Table 1.

Therapeutic Effect against Sarcoma 180 Tumor:

Five male ddY-strain mice each weighing 18 to 20 g were used for each group as test animals, and $5 \times 10^5$ Sarcoma 180 tumor cells were implanted subcutaneously into the animals at the axilla. One day after the implantation, 0.2 ml of physiological saline containing a test compound at the concentration indicated in Table 1 was intravenously administered to each mouse. T/C [T: average tumor volume ($mm^3$) of the group treated with the test compound, C: that of the control group which received an intravenous administration of 0.2 ml of physiological saline] was determined seven days after the implantation.

The results are shown in Table 1.

Table 1

| Compound No. | $HeLaS_3$ | Sarcoma 180 | |
|---|---|---|---|
| | $IC_{50}$ (nM) | Dose (mg/kg) | T/C |
| (I) | 31 | 0.70 | 0.095 |
| (III) | 16 | 0.50 | 0.14 |

Compound (I) showed an excellent activity equally to Compound (III).

Antibacterial activity:

Minimum inhibitory concentrations (MIC) of Compound (I) against various bacteria were determined by the agar dilution method (pH 7.0).

The results are shown in Table 2.

Table 2

| Test Organisms | MIC (μg/ml) |
|---|---|
| | Compound (I) |
| Staphylococcus aureus 209-P | 6.25 |
| Staphylococcus aureus 6538-P | 6.25 |
| Staphylococcus epidermidis S1318 | 6.25 |
| Bacillus subtilis ATCC6633 | 6.25 |
| Micrococcus luteus ATCC9341 | 25 |

The stability in the process for preparing a freezedried preparation of Compound (I) is described below by referring to Experimental Example 2.

Experimental Example 2

In 1 ml of distilled water was dissolved 50 mg of lactose and 0.5 mg of citric acid. Compound (I) or Compound (III) was dissolved in the solution to a concentration of 1 mg/ml. The test solution was poured in a vial, and kept in a thermostat at 25°C. The concentration of Compound (I) or Compound (III) in the solution was determined by High Performance Liquid Chromatography (HPLC) with the elapse of time. The concentration of each test compound in the test solution just after the dissolution was defined as 100 %. The residual rate (%) was calculated according to the following equation.

Residual Rate (%)

$$= \frac{\text{Concentration of test compound (t*=3 or 6)}}{\text{Concentration of test compound (t*=0))}} \times 100$$

*t means a time (hour).

The results are shown in Table 3.

Table 3

| Compound | Time (hr) | Residual Rate (%) |
|---|---|---|
| (I) | 0 | 100 |
| | 3 | 99 |
| | 6 | 98 |
| (III) | 0 | 100 |
| | 3 | 97 |
| | 6 | 95 |

The foregoing results reveal that Compound (I) has an improved stability in solution and can be advantageously used in the process for producing preparations.

Compound (I) may be used as anti-tumor agent , singly or in combination with at least one pharmaceutically acceptable carrier. For example, Compound (I) is dissolved in an aqueous solution of citric acid, glucose, lactose, mannitol, or the like to prepare a pharmaceutical composition suitable for injection. Alternatively, Compound (I) is freezedried in a conventional manner and a powder for the purpose of injection is prepared by adding sodium bromide thereto. If necessary, the pharmaceutical composition may contain additives well known in the field of medical preparation, for example, pharmaceutically acceptable salts.

Although the dose of the composition may vary depending upon the age, condition, etc. of the patient, it is suitable to administer Compound (I) in a dose of 0.01 to 5 mg/kg/day for mammals including human beings. Administration may be made, for example, once a day (single administration or consecutive

administrations) or intermittently 1 to 3 times a week or once every 2 to 3 weeks, intravenously. If desired, intraarterial administration, intraperitoneal administration, intrathoracical administration, etc. are also possible in a similar dose and in a similar manner.

The anti-tumor compositions of the present invention are expected to be effective against leukemia, gastric cancer, colon cancer, lung cancer, breast cancer, uterine cancer, etc. in mammals including human beings.

Certain specific embodiment of the present invention is illustrated by the following example.

The physicochemical properties of the compound shown in the following example were determined with the following equipment.

NMR Bruker AM-400 (400 MHz)
IR Japan Spectral Co., Ltd. IR-810

Example 1 Synthesis of Compound (I)

In 60 ml of acetone was dissolved 6.69 g (9.57 mmol) of Compound (II) (Japanese Published Unexamined Patent Application No. 128379/91), and 270 ml of methanol and 1.7 ml of 48% hydrobromic acid were added to the solution, followed by stirring at room temperature for one hour and further by stirring at 0 °C for 4 hours. The reaction mixture was filtered under reduced pressure and dried to give 6.42 g (yield 86%) of Compound (I).

The physicochemical properties of Compound (I) are as follows.

$^1$H-NMR (400 MHz, DMSO-$d_6$) $\delta$ (ppm); 11.97 (1H, s), 11.30 (1H, d, J = 1.9 Hz), 9.81 (1H, br), 7.94 (1H, s), 7.00 (1H, d, J = 2.1 Hz), 6.97 (1H, s), 4.65 (1H, dd, J = 10.5, 8.5 Hz), 4.47 (3H, m), 4.20 (1H, br), 3.94 (3H, s), 3.85 (3H, s), 3.82 (3H, s), 3.80 (3H, s), 3.53 (3H, br), 3.41 (1H, dd, J = 9.0, 9.0 Hz), 3.26 (4H, br), 2.89 (3H, br s), 2.69 (3H, s).

IR (KBr) $\nu$ ($cm^{-1}$); 1717, 1692, 1608, 1525, 1490, 1409, 1310, 1218, 1167, 1108.

| Elementary analysis: $C_{32}H_{36}BrN_5O_8 \cdot HBr \cdot H_2O$ | | | |
|---|---|---|---|
| Calculated (%): | C 47.92 | H 5.06 | N 8.73. |
| Found (%): | C 47.92 | H 5.10 | N 8.49. |

**Claims**

1. A compound represented by formula (I):

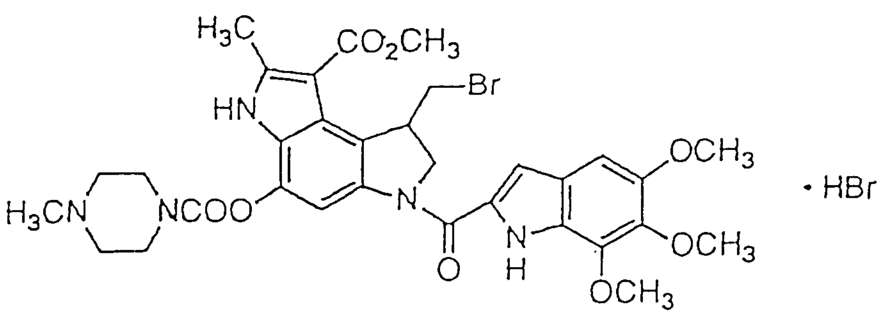

(I)

2. A pharmaceutical composition comprising a pharmaceutical carrier, and as an active ingredient, an effective amount of the compound defined by Claim 1.

3. The compound according to Claim 1 usable as a medicament.

4. Use of a compound according to Claim 1 or of a composition according to Claim 2 for the manufacture of a medicament having anti-tumor activity.

European Patent Office

# EUROPEAN SEARCH REPORT

Application Number

EP 92 11 6898

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 406 749 (KYOWA HAKKO KOGYO KABUSHIKI KAISHA)<br>* claims 1,14; examples 16,18; table 1 *<br>--- | 1,4 | C07D487/04<br>A61K31/495<br>//(C07D487/04, 209:00,209:00) |
| P,A | EP-A-0 468 400 (KYOWA HAKKO KOGYO CO.)<br>* page 3, line 1 - page 3, line 4; claim 1 *<br>----- | 1,4 | |
| | | | TECHNICAL FIELDS SEARCHED (Int. Cl.5) |
| | | | C07D<br>A61K |

The present search report has been drawn up for all claims

| Place of search | Date of completion of the search | Examiner |
|---|---|---|
| THE HAGUE | 11 JANUARY 1993 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)